# EUROPEAN PATENT SPECIFICATION

(11) **EP 1 148 065 B1**
(45) Date of publication and mention of the grant of the patent: **02.01.2008**
(21) Application number: 00108691.7
(22) Date of filing: 21.04.2000
(51) Int. Cl.: C07K 14/705, C07K 14/715, C07K 19/00, A61K 38/17

(54) **Fusion proteins comprising two soluble gp130 molecules**
Fusionsproteine, die zwei lösliche gp130 Moleküle enthalten
Protéines de fusion comprenant deux molécules gp130 solubles

(43) Date of publication of application: 24.10.2001
(73) Proprietor: CONARIS research institute AG, 24118 Kiel (DE)
(72) Inventor: Rose-John, Stefan, 24098 Kiel (DE)
(74) Representative: Schüssler, Andrea

(56) References cited:
- US-A- 5 426 048
- US-A- 5 783 672
- PAONESSA G ET AL: "TWO DISTINCT AND INDEPENDENT SITES ON IL-6 TRIGGER GP 130 DIMER FORMATION AND SIGNALLING" EMBO JOURNAL,GB,OXFORD UNIVERSITY PRESS, SURREY, vol. 14, no. 9, 1 May 1995 (1995-05-01), pages 1942-1951, XP000565718 ISSN: 0261-4189
- FISCHER M ET AL: "A BIOACTIVE DESIGNER CYTOKINE FOR HUMAN HEMATOPOIETIC PROGENITOR CELL EXPANSION" NATURE BIOTECHNOLOGY,US,NATURE PUBLISHING, vol. 15, no. 2, 1 February 1997 (1997-02-01), pages 142-145, XP002047603 ISSN: 1087-0156
- JOSTOCK T ET AL: "Immunoadhesins of interleukin-6 and the IL-6/soluble IL-6R fusion protein hyper-IL-6" JOURNAL OF IMMUNOLOGICAL METHODS,NL,ELSEVIER SCIENCE PUBLISHERS B.V.,AMSTERDAM, vol. 223, no. 2, 4 March 1999 (1999-03-04), pages 171-183, XP004158716 ISSN: 0022-1759
- TAGA T.; KISHIMOTO T.: 'GP130 AND THE INTERLEUKIN-6 FAMILY OF CYTOKINES' ANNUAL REVIEW OF IMMUNOLOGY vol. 15, 1997, pages 797 - 819

## Description

### FIELD OF THE INVENTION

The present invention relates to a pharmaceutical composition containing a fusion molecule comprising two soluble gp130 (sgp13C) molecules. In particular, an Fc fusion protein of soluble gp130 (sgp130-Fc) has been generated and has shown in several cell culture models as well as with lamina propria cells from Crohn's disease patients to specifically block IL-6 responses dependent on soluble IL-6R, whereas responses via the membrane bound IL-6R remain unaffected.

Additionally, sgp130-Fc also inhibited proliferation induced by leukemia inhibitory factor (LIF) and Oncostatin M (OSM).

### BACKGROUND OF THE INVENTION

The pleiotropic cytokine interleukin-6 (IL-6) shows a wide spectrum of biological functions among which stimulation of B cells and induction of acute phase protein synthesis in liver are mostly notable. IL-6 exerts its activity on target cells via binding to an IL-6 specific surface receptor ("IL-6R" or "gp80"). This receptor/ligand complex facilitates homodimerization of gp130 the second subunit of the IL-6 receptor complex.

Dimerization of gp130 results in transduction of an IL-6 signal (Taga et al., 1997a) Soluble forms of the IL-6R (sIL-6R) which are generated by two mechanisms alternative splicing and shedding are also able to trigger gp130 dimerization and signaling when complexed with IL-6 (Taga et al., 1997a as well as Rose-John et al., 1994)

Since the cytoplasmic portion of the IL-6R does not contribute to signal transduction (Taga et al., 1989), signaling by a gp130 homodimer can be induced by IL-6 in complex with membrane bound or soluble IL-6R (Taga et al., 1997b).

The presence of sIL-6R, however, leads to sensitization of IL-6 responsive cells towards the ligand, as described previously for human hepatoma cells HepG2 (Peters et al., 1996). Furthermore, it has been shown that strictly IL-6 dependent hybridoma cells do not proliferate in response to very low amounts of IL-6 when sIL-6R present in culture media is continuously removed (Galliard et al., 1997).

Initially described as the interleukin-6 signal transducer, gp130 is a transducer chain shared by many cytokines, such as IL-6, IL-11, leukemia inhibitory factor (LIF), oncostatin M (OSM), and ciliary neurotrophic factor (CNTF). All of these cytokines act via a bi- or tripartite receptor complex in which signaling is triggered by homodimerization (for IL-6) or heterodimerization with LIF-Rb/gp190 protein (for IL-11, LIF, OSM, and CNTF) of gp130. These cytokines thus mediate similar biologic activities in various tissues.

While gp130 can be found on nearly all cell types, the IL-6R shows a much more restricted expression. The release of sIL-6R by one cell type renders other cells, which only express gp13C responsive to IL-6. This scenario is called trans-signaling (Rose-John et al., (1994). Indeed, several cellular activities have been described which require the complex of sIL-6R and IL-6 and are not seen with IL-6 alone.

Soluble gp130 protein is found in high concentrations in human plasma (Narazaki et al., 1993).

Recently the designer-cytokine hyper-IL-6 (H-IL-6), in which the COOH-terminus of sIL-6R is covalently fused to the NH₃-terminus of mature IL-6 by a flexible peptide linker, has been described. As seen with the complex of IL-6/sIL-6R, H-IL-6 also acts on cells which only express gp130. In contrast to the separate components IL-6 and sIL-6R, a 100 to 1000 lower concentration of this fusion molecule is sufficient to induce comparable biological signals (Fischer et al., 1997).

WO 99/02552 describes chimeric proteins constructed from the fusion of the naturally occurring form of sIL-6R and IL-6, which are useful in the treatment of IL-6 related disorders.

One embodiment of WO 95/113C3 describes heterodimeric proteins comprising the extracellular domain of a cytokine receptor, such as sIL-6R or sCNTFR and the extracellular domain of gp130. Such heterodimers are reported to be effective antagonists of IL-6 or CNTF, because they would act as traps for IL-6 or CNTF, thus rendering the cytokine inaccessible to form a signal-transducing complex with the native membrane-bound forms of their receptors. US-Patent No. 5,783,672 and 5,426,048 describe the generation of heterodimeric receptor proteins comprising OSM-Rβ+gp130 and LIF-R+ gp130, respectively.

The extracellular portions of many cytokine receptors such as both TNF receptors (Van Zee et al., 1992) have been fused to the constant region of an immunoglobulin heavy chain (Fc). Thereby, disulfide-linked receptor homodimers are generated which are similar to IgG antibodies. Such fusion proteins are called Fc proteins or immunoadhesins. They show increased affinity to their ligands and an increased plasma half-life compared with the monomeric soluble receptors (for review Ashkenazi et al., 1997). Furthermore, biologically active Fc fusion proteins of IL-2, IL-10, IL-15 and IL-17 have been described. Similar to antibodies, secreted recombinant Fc fusion proteins can be purified from culture medium by single step purification via Protein A or Protein G Sepharose.

### DESCRIPTION OF THE INVENTION

The aim of the present invention is to provide a pharmaceutical composition containing a fusion molecule comprising two soluble gp130 (sgp130) molecules. In particular, a Fc fusion protein of soluble gp130 (sgp130-Fc) has been generated and has been found to be highly expressed in COS-7 cells. This fusion protein has shown in several cell culture models as well as with lamina propria cells from Crohn's disease patients to specifically block IL-6 responses dependent on soluble IL-6R, whereas responses via the membrane bound IL-6R remain unaffected. Additionally, sgp130-Fc also inhibited proliferation induced by leukemia inhibitory factor (LIP) and Oncostatin M (OSM) albeit at about 1000 fold higher concentrations.

The fusion molecules of pharmaceutical composition of the present invention may be engineered using known methods. The domains utilized may consist of the entire extracellular domain of gp130 or they may consist of mutants or fragments thereof that maintain the ability to inhibit the activity of the agonistic complex IL-6/sIL-6R.

The cloning and expression of human gp130 has been reported in 1990 together with its putative extracellular domain (Hibi et al., 1990).

A splice variant of the cDNA encoding a soluble form of gp130 has been found expressed in blastocysts (Sharkey et al., 1995). Such variant lacks the intracellular signaling domain. Fusion molecules according to the invention and comprising two of such sgp130 variants are comprised in the scope of the present invention.

The fusions may be direct (the C-terminus of one polypeptide chain is linked to the N-terminal of the other through a simple covalent bond) or they may employ a flexible linker domain, such as the hinge region of human IgG, or polypeptide linkers consisting of small amino acids such as glycine, serine, threonine or alanine, at various lengths and combinations. Additionally, the fusion molecules of the invention may be tagged by His-His-His-His-His-His (His6), to allow rapid purification by metal-chelate chromatography, and/or by epitopes to which antibodies are available, to allow for detection on western blots, immunoprecipitation, or activity depletion blocking in bioassays.

In another embodiment, the fusion protein of the pharmaceutical composition of the invention is prepared using a similar method, but using the Fc-domain of human IgG1 (Aruffo et al., 1991). In this case homodimeric molecules carrying the Fc-domain will be expressed as disulfide-linked homodimers.

In another embodiment of the pharmaceutical composition of the invention the fusion molecules of the invention are prepared by expression as fusion molecules utilizing flexible linker loops. A DNA construct encoding the fusion protein is designed such that it expresses two soluble or extracellular domains fused together in tandem ("head to head") by a flexible loop. This loop may be entirely artificial (e.g. polyglycine repeats interrupted by serine or threonine at a certain interval) or "borrowed" from naturally occurring proteins (e.g. the hinge region of hIgG).

Moreover, the present invention also contemplates the use of engineered, mutated versions of gp130 with novel properties that allow to bind the agonistic complex IL-6/sIL-6R..

A variety of means can be used to generate and identify mutations of sgp130 that have the desired properties. Random mutagenesis by standard methods of the DNA encoding sgp130 may be used, followed by analysis of the collection of products to identify mutated cytokines having the desired novel properties as outlined below. Mutagenesis by genetic engineering has been used extensively in order to elucidate the structural organization of functional domains of recombinant proteins. Several different approaches have been described in the literature for carrying out deletion or substitution mutagenesis. The most successful appear to be alanine scanning mutagenesis (Cunningham et, (1989a) and homolog-scanning mutagenesis (Cunningham et al., 1989b).

The fusion molecules of the pharmaceutical composition of the present invention may be prepared by cloning and expression in a prokaryotic or eukaryotic expression system, using the appropriate expression vectors. Any method known in the art can be employed.

For example the DNA molecules coding for the proteins of the invention are inserted into appropriately constructed expression vectors by techniques well known in the art (see Sambrook et al, 1989). Double stranded cDNA is linked to plasmid vectors by homopolymeric tailing or by restriction linking involving the use of synthetic DNA linkers or blunt-ended ligation techniques: DNA ligases are used to ligate the DNA molecules and undesirable joining is avoided by treatment with alkaline phosphatase.

In order to be capable of expressing the desired protein, an expression vector should comprise also specific nucleotide sequences containing transcriptional and translational regulatory information linked to the DNA coding the desired protein in such a way as to permit gene expression and production of the protein. First in order for the gene to be transcribed, it must be preceded by a promoter recognizable by RNA polymerase, to which the polymerase binds and thus initiates the transcription process. There are a variety of such promoters in use, which work with different efficiencies (strong and weak promoters).

For eukaryotic hosts, different transcriptional and translational regulatory sequences may be employed, depending on the nature of the host. They may be derived form viral sources, such as adenovirus, bovine papilloma virus, Simian virus or the like, where the regulatory signals are associated with a particular gene, which has a high level of expression. Examples are the TK promoter of the Herpes virus, the SV40 early promoter, the yeast gal4 gene promoter, etc. Transcriptional initiation regulatory signals may be selected which allow for repression and activation, so that expression of the genes can be modulated.

The DNA molecule comprising the nucleotide sequence coding for the fusion molecule of the invention is inserted into vector(s), having the operably linked transcriptional and translational regulatory signals, which is capable of integrating the desired gene sequences into the host cell. The cells which have been stably transformed by the introduced DNA can be selected by also introducing one or more markers which allow for selection of host cells which contain the expression vector. The marker may also provide for phototrophy to an auxotropic host, biocide resistance, e.g. antibiotics, or heavy metals such as copper, or the like. The selectable marker gene can either be directly linked to the DNA gene sequences to be expressed, or introduced into the same cell by co-transfection. Additional elements may also be needed for optimal synthesis of proteins of the invention.

Factors of importance in selecting a particular plasmid or viral vector include: the ease with which recipient cells, that contain the vector may be recognized and selected form those recipient cells which do not contain the vector; the number of copies of the vector which are desired in a particular host; and whether it is desirable to be able to "shuttle" the vector between host cells of different species.

Once the vector(s) or DNA sequence containing the construct(s) has been prepared for expression the DNA construct(s) mat be introduced into an appropriate host cell by any of a variety of suitable means: transformation, transfection, conjugation, protoplast fusion, electroporation, calcium phosphate-precipitation, direct microinjection, etc.

Host cells may be either prokaryotic or eukaryotic. Preferred are eukaryotic hosts, e.g. mammalian cells, such as human, monkey, mouse, and Chinese hamster ovary (CHO) cells, because they provide post-translational modifications to protein molecules, including correct folding or glycosylation at correct sites. Also yeast cells can carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products and secretes peptides bearing leader sequences (i.e., pre-peptides).

After the introduction of the vector(s), the host cells are grown in a selective medium, which selects for the growth of vector-containing cells. Expression of the cloned gene sequence(s) results in the production of the desired proteins.

Purification of the recombinant proteins is carried out by any one of the methods known for this purpose, i.e. any conventional procedure involving extraction, precipitation, chromatography, electrophoresis, or the like. A further purification procedure that may be used in preference for purifying the protein of the invention is affinity chromatography using monoclonal antibodies which bind the target protein and which are produced and immobilized on a gel matrix contained within a column. Impure preparations containing the recombinant protein are passed through the column. The protein will be bound to the column by the specific antibody while the impurities will pass through. After washing, the protein is eluted from the gel by a change in pH or ionic strength.

A further object of the present invention is a DNA molecule comprising the DNA sequence coding for the above fusion protein, as well as nucleotide sequences substantially the same. "Nucleotide sequences substantially the same" includes all other nucleic acid sequences which, by virtue of the degeneracy of the genetic code, also code for the given amino acid sequence.

For the production of the pharmaceutical composition of the fusion protein of the invention, the DNA sequences are obtained from existing clones.

Analogs or variants in accordance with the present invention may also be determined in accordance with the following procedure. The DNA of the native sequences is known in the prior art and is found in the literature. Polypeptides encoded by any nucleic acid, such as DNA or RNA, which hybridizes to the complement of the native DNA or RNA under highly stringent or moderately stringent conditions, as long as that polypeptide maintains the biological activity of the native sequence, are also considered to be within the scope of the present invention.

Stringency conditions are a function of the temperature used in the hybridization experiment, the molarity of the monovalent cations and the percentage of formamide in the hybridization solution. To determine the degree of stringency involved with any given set of conditions, one first uses the equation of Meinkoth et al. (1984) for determining the stability of hybrids of 100% identity expressed as melting temperature Tm of the DNA-DNA hybrid: Tm = 81.5°C + 16.6 (_{Log}M) + 0.41 (%GC) - 0.61 (% form) - 500/L, where M is the molarity of monovalent cations, %GC is the percentage of G and C nucleotides in the DNA, % form is the percentage of formamide in the hybridization solution, and L is the length of the hybrid in base pairs. For each 1°C that the Tm is reduced from that calculated for a 100% identity hybrid, the amount of mismatch permitted is increased by about 1%. Thus, if the Tm used for any given hybridization experiment at the specified salt and formamide concentrations is 10°C below the Tm calculated for a 100% hybrid according to equation of Meinkoth, hybridization will occur even if there is up to about 10% mismatch.

As used herein, highly stringent conditions are those which are tolerant of up to about 15% sequence divergence, while moderately stringent conditions are those which are tolerant of up to about 20% sequence divergence. Without limitation, examples of highly stringent (12-15°C below the calculated Tm of the hybrid) and moderately (15-20°C below the calculated Tm of the hybrid) conditions use a wash solution of 2 X SSC (standard saline citrate) and 0.5% SDS at the appropriate temperature below the calculated Tm of the hybrid. The ultimate stringency of the condition is primarily due to the washing conditions, particularly if the hybridization conditions used are those which allow less stable hybrids to form along with stable hybrids. The wash conditions at higher stringency then remove the less stable hybrids. A common hybridization condition that can be used with the highly stringent to moderately stringent wash conditions described above is hybridization in a solution of 6 X SSC (or 6 X SSPE), 5 X Denhardt's reagent, 0.5% SDS, 100 µg/ml denatured, fragmented salmon sperm DNA at a temperature approximately 20° to 25°C below the Tm. If mixed probes are used, it is preferable to use tetramethyl ammonium chloride (TMAC) instead of SSC (Ausubel, 1987-1998).

Another object of the present invention are pharmaceutical compositions containing PEGylated or other chemically modified forms of the hybrid proteins.

The fusion molecules of the pharmaceutical composition of the present invention are useful in the treatment and/or prevention of all the pathologies, in which the activity of the agonistic complex IL-6/sIL-6R must be inhibited. For example, therapeutic uses of the fusion molecules would include the following:
a) IL-6 appears to be directly involved in multiple myeloma by acting in either an autocrine or paracrine fashion to promote tumor formation (van Oers et al., 1993). Furthermore, the elevated IL-6 levels create undesirable secondary effects such as bone resorption, hypercalcemia, and cachexia. In these cases it is known that sIL-6R sensitizes target cells for IL-6. Therefore, the fusion molecules of the invention as described herein would be beneficial for both the secondary effects as well as for inhibiting tumor growth.
b) In autoimune diseases: the pathogenic significance of IL-6 in autoimmune disorders has been reviewed by many authors in the literature (see Yoshizaki et al., 1992), thus interference with IL-6 signal transduction may be useful for autoimmune disease therapy (Nishimoto et al., 1999). Examples of such pathologies are systemic lupus erythematosus, Hashimoto's thyroiditis, scleroderma, rheumatoid arthritis, multiple sclerosis, Autoimmune epithelitis, Diabetes mellitus, Sjögren's syndrome, polymyositis, glomerulonephritis and other inflammatory diseases, such as psoriasis and Chron's disease.
c) In osteoporosis, which can be exacerbated by lowering of estrogen levels in postmenopausal women or through ovariectomy, IL-6 appears to be a critical mediator of osteoclastogenesis, leading to bone resorption (Jilka et al., 1992). Importantly, IL-6 only appears to play a major role in the estrogen-depleted state, and apparently is minimally involved in normal bone maintenance. Consistent with this, experimental evidence indicates that function-blocking antibodies to IL-6 can reduce the number of osteoclasts [Jilka et al., 1992)]. While estrogen replacement therapy is also used, there appear to be side effects that may include increased risk of endometrial and breast cancer. Thus, the fusion molecules of the invention as described herein would be more specific to reduce osteoclastogenesis to normal levels.
d) IL-6 may be a mediator of tumor necrosis factor (TNP) that leads to cachexia associated with AIDS and cancer (Strassmann et al., 1992), perhaps by reducing lipoprotein lipase activity in adipose tissue (Greenberg, et al., 1992). Accordingly, the fusion molecules of the invention described herein would be useful in alleviating or reducing cachexia in such patients.
e) bacterial and viral infections: the presence of Human Herpes Virus 8 (HHV8) has been demonstrated in more than 90 % of Kaposi's sarcoma (KS) lesions. Moreover, the virus has been identified in primary effusion lymphoma (PEL) and in patients with multicentric Castleman disease (MCD). Intriguingly, bone marrow dendritic cells from multiple myeloma (MM) patients were shown to be infected by HHV8. Since then, the association of HHV8 with MM has been a subject of fierce debate, which was recently revived. The genome of HHV8 codes for several proteins with significant homologies to human antiapoptotic proteins, chemokines, and cytokines including a viral form of Interleukin-6 (vIL-6) with 25 % homology to human IL-6 (Moore et al., 1996).

vIL-6 has been demonstrated to have biologic activities reminiscent of human IL-6, i.e. stimulation of proliferation of murine hybridoma and human myeloma cells. More recently it was shown in mice, injected with vIL-6 transfected NIH3T3 cells, that vIL-6 induced angiogenesis and hematopoiesis. It was concluded that through these functions vIL-6 played an important role in the pathogenesis of HHV8-associated disorders. The contribution of the IL-6R to vIL-6 signaling has been discussed controversially. Molden et al. using unpurified supernatants of vIL-6 transfected COS-7 cells have shown that STAT activity was induced in cells expressing gp130 but no IL-6R (Molden et al., 1997). In contrast, Burger et al. found that the activity of vIL-6 was reduced by an IL-6 receptor antagonist, arguing for an involvement of IL-6R in vIL-6 signaling (Burger et al., 1998). Here we show that purified recombinant vIL-6 physically interacted with gp130 but not with IL-6R and that IL-6R was dispensable for the biologic activity of vIL-6. The activity of vIL-6 is inhibited by sgp130-Fc fusion protein.

Pharmaceutical compositions according to the invention include the fusion molecules described above in a pharmacologically acceptable carrier, linked to a carrier and/or incorporated into liposomes, microcapsules, and controlled release preparation. For example, the pharmaceutical composition may comprise one or more of the fusion molecules in an aqueous solution, such as sterile water, saline, phosphate buffer or dextrose solution. Alternatively, the active agents may be comprised in a solid (e.g. wax) or semisolid (e.g. gelatinous) formulation that may be implanted into a patient in need of such treatment.

The administration route may be any mode of administration known in the art, including but not limited to intravenously, intrathecally, subcutaneously, by injection into involved tissue, intraarterially, intranasally, orally, or via an implanted device. Administration may result in the distribution of the active agent of the invention throughout the body or in a localized area. For example, in some conditions, which involve distant regions of the nervous system, intravenous or intrathecal, administration of agent may be desirable. In some situations, an implant containing active agent may be placed in or near the lesioned area.

Another object of the present invention is, therefore, the method for treating and/or preventing one of the above-mentioned diseases by an effective amount of the fusion molecule of the invention, together with a pharmaceutically acceptable excipient.

An "effective amount" refers to an amount of the active ingredients that is sufficient to affect the course and the severity of the disease, leading to the reduction or remission of such pathology. The effective amount will depend on the route of administration and the condition of the patient.

Effective doses useful for treating and/or preventing these or other related diseases or disorders may be determined using methods known to one skilled in the art (see for example, Fingl, et al., The Pharmacological Basis of Therapeutics, Goodman and Gilman, eds. Macmillan Publishing Co., New York, pp. 1-46 ((1975)).

A further object of the present invention are the pharmaceutical compositions containing the fusion molecule of the invention, in the presence of one or more pharmaceutically acceptable excipients.

"Pharmaceutically acceptable" is meant to encompass any carrier, which does not interfere with the effectiveness of the biological activity of the active ingredient and that is not toxic to the host to which is administered.

The invention will now be described by means of the following Examples, which should not be construed as in any way limiting the present invention. These Examples will refer to the Figures specified here below.

### DESCRIPTION OF THE FIGURES

Figure 1
   Schematic drawings of sgp130-Fc and sgp130his constructs.
Figure 2
   Interaction of gp130 proteins with the IL-6/slL-6R complex. (A) Metabolically labeled H-IL-6 was immunoprecipitated from 200 µl culture medium of transfected COS-7 cells using 0.5 g of sgp130-Fc (lane 2) or an irrelevant Fc fusion protein (R27080Fc) as negative control (lane 1). (B) Metabolically labeled sgp130his was immunoprcipitated from 200 µ culture medium of transfected COS-7 cells using 0.5 µg of H-IL-6-Fc (lane 2) or the unrelated R27080Fc protein as negative control (lane 1). Immunocomplexes were analyzed by SDS-PAGE and fluorography.
Figure 3
   Binding of human and murine gp130 to IL-6/IL-6R complex components: Metabollically labeled murine slL-6R (lane 1-7) or human slL-6R (lane 8-14) was immunoprecipitated from 250 µl culture media of transfected COS-7 cells using either 1 µg of human sgp130-Fc (h), murine gp130Fc (m) or human IL-6Fc (h), as indicated in the panel. Precipitation took place in the presence or absence of 350 ng of human IL-6 (h) or murine IL-6 (m), as indicated. Immunocomplexes were analyzed by SDS-PAGE and fluorography.
Figure 4
   Kinetics of binding of H-IL-6 to immobilized sgp130-Fc. (A) Association of H-IL-6 to immobilized sgp130-Fc led to the increase of the resonance angle alpha as a function of time. The association phase was recorded for different nanomolar H-IL-6 concentrations as indicated. (B) Calculation of the obtained data from the curves allowed the determination of the dissociation rate constant kd from the ordinate intercept and the association rate constant ka from the slope of the graph. All kinetic experiments were performed at controlled temperature (25 °C).
Figure 5
   (A) Proliferation of BAF/gp130 cells in response to 5 ng/ml H-IL-6 and increasing amounts of sgp130-Fc or sgp130his. (B) Proliferation of BAF/gp130/IL-6R cells in response to 1 ng/ml IL-6 and increasing amounts of sgp130-Fc or sgp130his. The data represent means +/- SD of two parallel experiments. (C) Proliferation of BAF/gp130 cells in response to 100 ng/ml IL-6 together with 50 ng/ml slL-6R and increasing amounts of sgp130-Fc or 10 ng/ml H-IL-6 and increasing amounts of sgp130-Fc. The data represent means +/- SD of three parallel experiments.
Figure 6
   Proliferation of BAF/gp130/LIFR/CNTFR cells in response to 10 ng/ml H-IL-6, 1 ng/ml LIF, 10 ng/ml OSM or 0.5 ng/ml CNTF in the presence of increasing amounts of sgp130-Fc. The data represent means +/- SD of two parallel experiments.
Figure 7
   Inhibition of acute phase in hepatoma cells: (A) HepG2 cells were stimulated with 100 ng/ml of IL-6 or H-IL-6 for 18 h in the presence or absence of 1 µg/ml sgp130-Fc, (B) HepG2/IL-6 cells were stimulated with 100 ng/ml of IL-6R-Fc or H-IL-6 with or without 1 µg/ml sgp130-Fc for 18 h. Unstimulated cells treated with or without sgp130-Fc were used as control (co). α1-Antichymotrypsin expression of the cells was analyzed by 4 h of pulse labeling and subsequent immuneprecipitation with a specific antibody. Immunocomplexes were analyzed by SDS-PAGE and fluorography.
Figure 8
   Analysis of apoptosis in lamina propria mononuclear cells (LPMC) of Crohn patient. LPMCs were isolated as described and cultured as described for 48 h in the presence or absence of 10 µg/ml of a neutralizing IL-6R specific antibody or sgp130-Fc. Subsequently cells were stained for Annexin V and propidiumiodide and analyzed by FACS. Percentage of apoptotic (Annexin V positive and propidiumiodide negative) cells is shown.

### EXAMPLES

### EXAMPLE 1

### Materials

DMEM, penicillin and streptomycin were purchased from Gibco (Eggenstein, Germany). FCS was obtained from Seromeal (Berlin, Germany). DEAE-Dextran was purchased from Sigma (Taufkirchen, Germany). Restriction enzymes were from New England Biolabs (Schwalbach, Germany) or AGS (Heidelberg, Germany). T4-DNA-Ligase and Polynucleotide Kinase were from Boehringer Mannheim (Mannheim, Germany). Protein A Sepharose was obtained from Pharmacia (Freiburg, Germany). Tran-35S-Label (44 TBq/mmol) was from ICN (Meckenheim, Germany), [6-3H]thymidine (74 GBq/mmol) was obtained from Amersham International (Aylesbury, UK.). X-Ray films (X-OMAT-AR) were from Eastman Kodak (Rochester, NJ). H-IL-6 was generated as described; H-IL-6, sIL-6R, H-IL-6-Fc and IL-6-Fc were produced as described (Fischer et al., 1997); Özbek et al., 1998; Jostock et al., 1999.

The human IL-6R-Fc was constructed by fusing the cDNA coding for the extracellular portion of the human IL-6R to a cDNA coding for the human Fc portion of IgG1 using the restriction sites Sspl and EcoRV, respectively. Human sIL-6R and murine sIL-6R cDNAs were generated as described previously (Mackiewicz et al., 1992; Mackiewicz et al., 1995). The pCDM8 vector (Invitrogen, Leek, NL) was used for expression of sIL-6R proteins in mammalian cells. H-IL-6 in the pCDM8 expression vector

### Construction of gp130 expression plasmids

The cDNA sequence coding for the extracel(ular domain of gp130 was fused to a Bgl2/Notl fragment coding the constant region of a human IgG1 heavy chain [Fanslow et al., 1992). A Xhol/EcoR1 fragment of the gp130 cDNA was ligated to the Fc portion via a synthetic oligonucleotide adapter. To obtain a poly His tagged version of soluble gp130 the Fc portion in the sgp130-Fc construct was replaced by a synthetic oligonucleotide adapter coding for 6 histidine residues followed by a translational stop codon. For expression of proteins the mammalian expression plasmid pDC409 (Giri et al., 1994) was used.

### Culture and transfection of cells

HepG2, COS-7, BAF/gp130 and BAF/gp130/IL-6R cells were grown in DMEM at 37 °C, 5 % CO2 in a water saturated atmosphere. Cell culture media were supplemented with 10 % FCS, 100 mg/I streptomycin and 60 mg/I penicillin. BAF/gp130/IL-6R were cultured in presence of 10 ng/ml IL-6, BAF/gp130 in presence of 10 ng/ml H-IL-6, BAF/gp130/LIFR/CNTFR in presence of 10 ng/ml CNTF. COS-7 cells were transfected using the DEAE-Dextran method (McMahan et al., 1991). For production of recombinant proteins cells were cultured in media containing 0.5 % FCS, supernatants were collected on day 5 post transfection.

### Purification of recombinant proteins

sgp130-Fc was purified as described for IL-6Fc previosly. sgp130his was purified from 900 mi culture media at a t mi NJ/chelate column (Pharmacia) by elution with 100 mM imidazol using an FPLC system (Aekta Explorer, Pharmacia). Protein containing fractions identified by SDS-PAGE and subsequent silverstainig were pooled and dialized against PBS. Concentrations of purified proteins were determined using a BCA micro protein assay (Pierce, Rockford, IL) following the manufacturer's instructions or estimated from silverstained SDS-PAGE.

### Immunoprecipitation of proteins

Cells were metabollically labeled 48 h after transfection with 50 mCi/mi [³⁵S]methionine/[³⁵S]cysteine in methionine/cysteine free medium for 8 hours. Secreted proteins were immunoprecipitated as using 1 µg/ml of appropriate antibodies or Fc proteins, Immune complexes were precipitated from culture media using Protein A Sepharose, analyzed by SDS-PAGE (Laemmli, 1970) and visualized by fluorography using the flourographic intensifyer solution "Amplify" (Amersham).

### Binding assay

sgp130-Fc was covalently immobilized to a carboxymethyl dextran matrix (Fisons, Loughborough, UK) following the manufacturer's recommendations (Krebs et al. 1998). Binding experiments were performed at controlled temperature (25 °C) with different concentrations of H-IL-6 using the IASYS^{™} (Fisons) optical biosensor. Association and dissociation affinograms were analyzed by nonlinear regression with the FASTfit (Fisons) software, which uses the Marquard-Levenburg algorithm for iterative data fitting.

### Proliferation assays

BAF/gp130, BAF/gp130/IL-6R and BAF/gp130/LIFR/CNTFR cells (Kallen et al., 1999) were extensively washed with PBS in order to remove growth factors and resuspended in cytokine free medium. 5 x 10³ cells/well of a 96-well plate were cultured in a final volume of 100 mi with cytokines and increasing amounts of gp130 proteins as indicated in figures for 68 hours and subsequently pulse labeled with 0.25 mCi [³H]thymidine for 4 hours. Cells were harvested on glass filters and incorporated [³H]thymidine was determined by scintillation counting.

### Acute phase assay on hepatoma cells

HepG2 cells and HepG2 cells stably transfected with human IL-6 cDNA (HepG2/IL-6 cells) (Mackiewicz et al., 1992) were grown near confluency and stimulated for 18 h with 100 ng/ml IL-6, H-IL-6 or sIL-6R-Fc in the presence or absence of 1 µg/ml sgp130-Fc as indicated in the figures.
Subsequently cells were pulse labeled for 4 h with [³⁵S]-cysteine/methionine (10 µCi/ml). The supernatants of the cells were treated with pansorbin for 1 h before radioactive labeled α1-antichymotrypsin was immunoprecipitated with an specific antibody (Gibco, Eggenstein, Germany) and detected by SDS-PAGE and fluorography.

### Isolation and stimulation of human lamina propria mononuclear cells

Lamina propria mononuclear cells (LPMC) were and cultured in complete media consisted of RPMI-1640 with 3 mM L-glutamine, 10 mM HEPES Buffer, 10 µg/ml gentamycin (Whittecker, Walkersville, MD), 100 U/mi each of penicillin and streptomycin (Whittacker), 0.05 mM 2ME (Sigma Chemicals, St. Louis, MO) and 10 % heat-inactivated fetal calf serum. Cells were stimulated with C reactive protein (10 ug/ml Sigma Chem), 50 ng/ml PMA and 10 ug/ml phytohaemagglutinin (PHA, Sigma) in the presence or absence of sgp130-Fc or an neutralizing IL-6R specific antibody, both at 1 and 10 µg/ml as indicated in the figure. After 48 h cells were stained with annexin V and propidium iodide using the Annexin V FITC Apoptosis Detection Kit I (Pharmingen) and analyzed by FACS.

### Results and discussion

### Expression and purification of soluble gp130 protein.

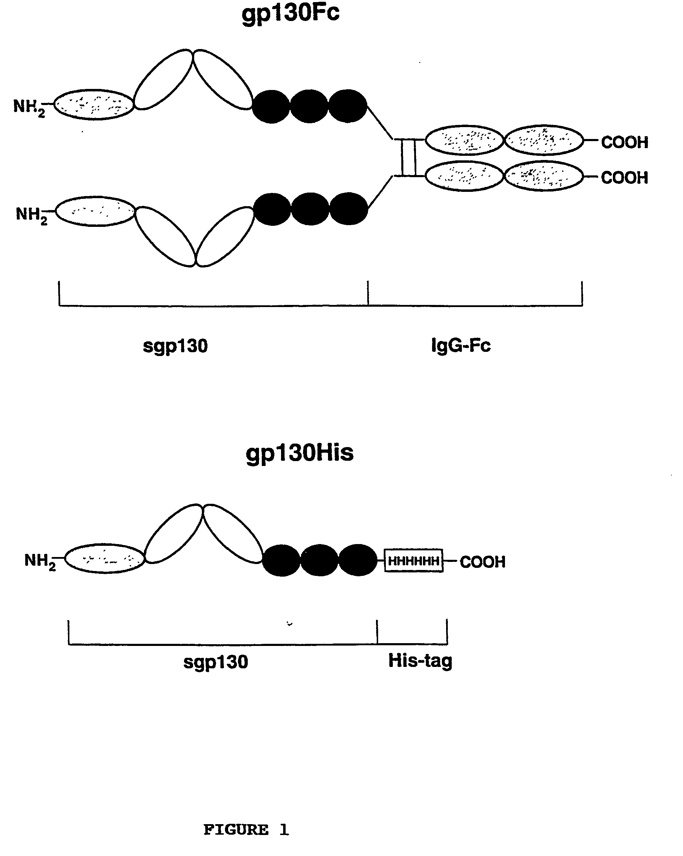

Two soluble forms of gp130 were generated. The cDNA coding the extracellular portion of human or murine gp130 was fused to the constant region of a human IgG1 heavy chain (sgp130-Fc). This kind of fusion proteins are generally called Fc-proteins or immunoadhesins (Ashkenazi et al., 1997; Jostok et al., 1999). A schematic representation of sgp130-Fc is shown in Fig. 1. In the second construct we added a "hexa-histidine" sequence tag to the COOH-terminus of the extracellular part of human gp130 (sgp130his) (Fig. 1). sgp130-Fc and sgp130his were transiently expressed in COS-7 cells and purified from culture media by affinity chromatography at Protein A Sepharose or Ni-Chelat Agarose, respectively. Purified gp130 proteins were analyzed by SDS-PAGE under reducing conditions and silver staining.

### Binding of sgp 130-Fc to the complex of IL-6 and soluble IL-6R.

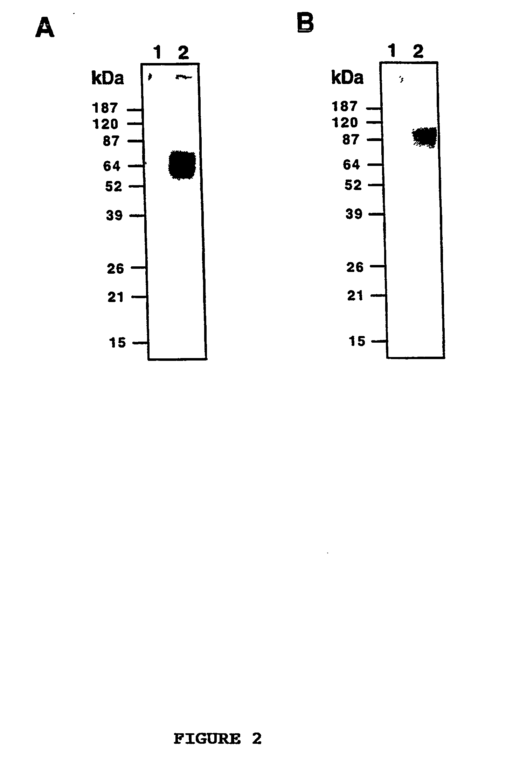

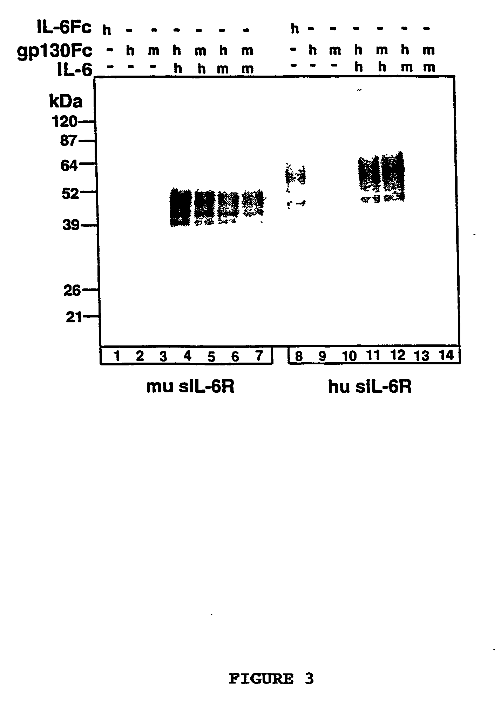

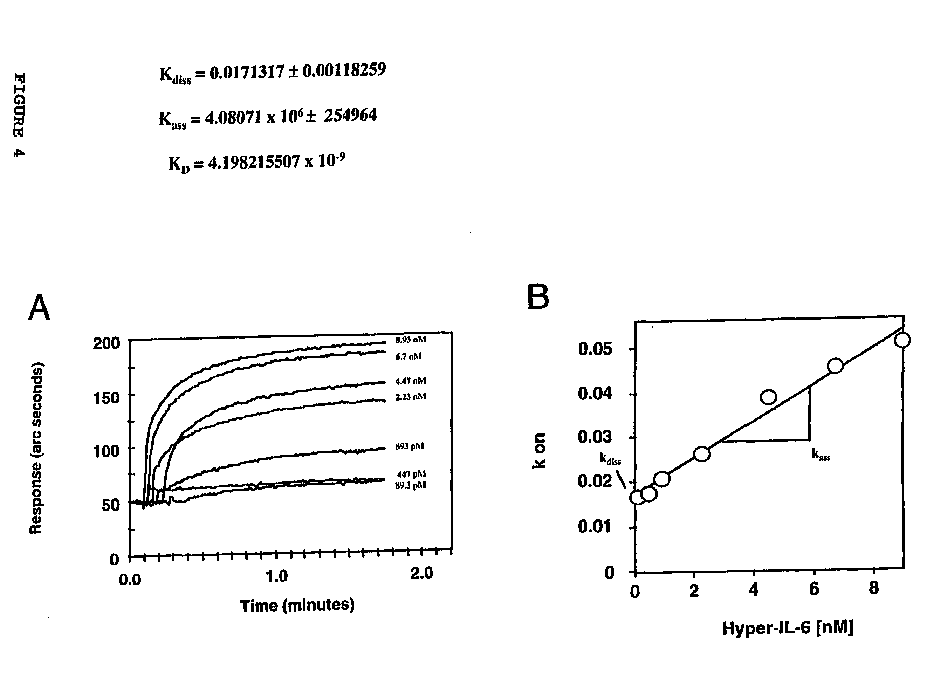

The formation of a ternary complex composed out of IL-6, the soluble forms of IL-6R and gp130 has been demonstrated previously. To analyze binding of sgp130-Fc and sgp130his to IL-6/sIL-6R we used the fusion protein "Hyper-IL-6" (H-IL-6) in which the COOH-terminus of the ligand binding domain of the IL-6R is fused to the NH2-terminus of IL-6 via a flexible peptide linker (Fischer et al., 1997). As shown in figure 2, radiolabeled H-IL-6 and sgp130his were immunoprecipitated with sgp130-Fc and H-IL-6Fc, respectively.
Human IL-6 binds the human sIL-6R and with lower affinity the murine sIL-6R, whereas murine IL-6 only binds the murine receptor. Therefore, it was of interest to investigate if further species restrictions apply to the formation of the ternary complex of IL-6, sIL-6R and sgp130 (Fig. 3). Radiolabeled murine or human sIL-6R was immuneprecipitated with murine or human sgp130-Fc in the absence or presence of murine or human IL-6. No sIL-6R was co-precipitated by sgp130-Fc in absence of IL-6. Murine sIL-6R formed a ternary complex with all combinations IL-6 and sgp130-Fc. As expected, human sIL-6R could only be co-precipitated by murine or human sgp130-Fc in the presence of human IL-6. Interestingly, the murine sIL-6R was not precipitated by IL-6Fc, a fusion protein of human IL-6 and human IgG1, indicating a very low affinity of human IL-6 to murine sIL-6R in the absence of gp130. Our results cleady show that human sIL-6R can interact with murine sgp130 and viceversa.
Kinetics of binding of H-IL-6 to sgp130-Fc were measured by plasmon resonance (Fig. 4A). sgp130-Fc was immobilized to an IASYS^{™} cuvette (see "Materials and Methods") and real-time interaction of H-IL-6 and sgp130-Fc was analyzed. In Fig. 4 association curves recorded for 7 concentrations of H-IL-6 are shown. The data obtained were used to calculate an association rate, kass = 4.08 x 106 +/- 2.55 x 105 M-1 s-1, and a dissociation rate, kdiss = 1.71 x 10-2 +/- 0.0012 s-l, between H-IL-6 and sgp130-Fc. From these values an affinity of Kd = 4.20 nM can be derived (Fig, 4B).

### Inhibition of sl£-61R dependent proliferation of BAF/3 cells.

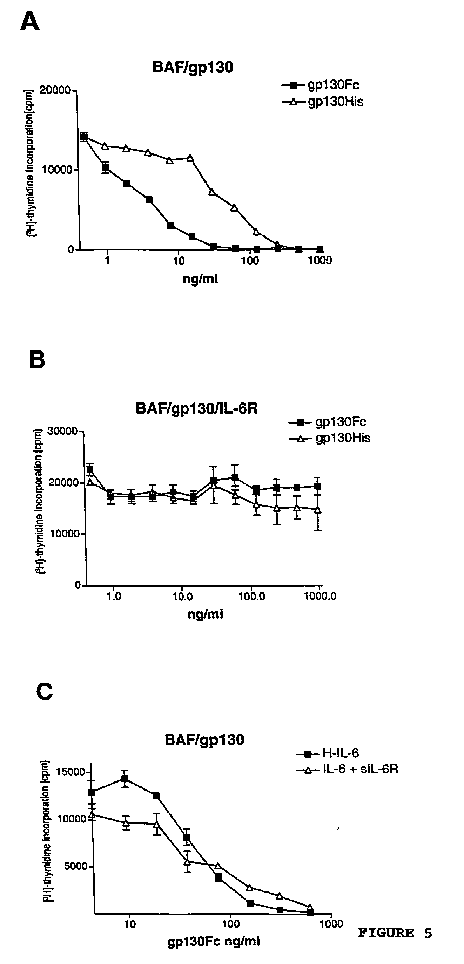

Having shown that sgp130 specifically interacts with the complex of IL-6/sIL-6R it was of interest to know if sgp130 proteins interfere with the biotogic activity of IL-6 and sIL-6R. We made use of the IL-3 dependent pre-B cells BAF/3 which do not express gp130 and are therefore not responsive to IL-6 or IL-6/sIL-6R. BAF/3 cells stable transfected with human gp130 cDNA (BAF/gp130) or human gp130 and sIL-6R cDNAs (BAF/gp130/IL-6R), however, in the absence of IL-3 grow in response to IL-6/sIL-6R or IL-6, respectively 0. Proliferation of BAF/gp130 cells stimulated by H-IL-6 was blocked by sgp130-Fc and sgp130his in a dose dependent manner (Fig. 5A). sgp130-Fc turned out to be about 10 fold more active than sgp130his, when of both proteins were used in equal molar concentration. None of the gp130 proteins had an influence on proliferation of BAF/gp130/IL-6R cells induced by IL-6 (Fig. 5B). These results clearly show that sgp130 specifically neutralizes the activity of sIL-6R complexed with IL-6 and fails to interfere with responses triggered via membrane bound IL-6R. Furthermore, we compared the effect of sgp130-Fc on BAF cell proliferation induced by H-IL-6 and IL-6 plus sIL-6R and obtained similar results. A concentration of 20 ng sgp130-Fc/mi led to 50% inhibition of proliferation induced by 10 H-IL-6 and 10 IL-6 plus 50 sIL-6R, respectively (Fig. 5C).

### Effect of sgp130-Fc on responses to other IL-6 type cytokines

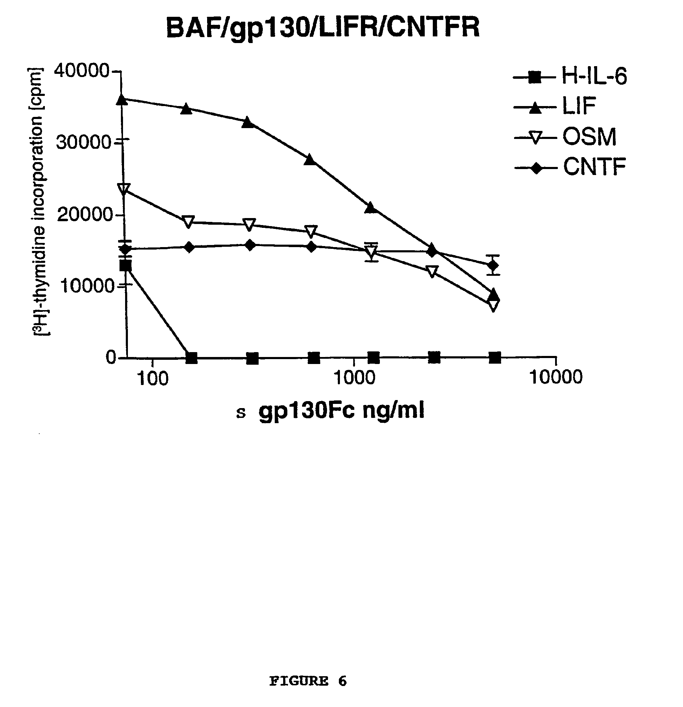

Besides being the signal transducing subunit of the IL-6R complex, gp130 is also involved in signaling in response to e.g. leukemia inhibitory factor (LIF), oncostatin M (OSM) and ciliary neurotrophic factor (CNTF). LIF and OSM act via a surface receptor composed of gp130 and LIFR. The CNTFR complex also contains gp130 and LIFR and, similar to the IL-6R complex, an additional ligand binding protein (CNTFR). To investigate the effect of sgp130-Fc on responses to these IL-6 type cytokines, we used BAF/3 cells expressing gp130, LIFR and CNTFR (BAF/gp130/LIFR/CNTFR). Whereas sgp130-Fc had no effect on CNTF induced proliferation of these cells, proliferation stimulated by LIF and OSM was inhibited at high concentrations of sgp130-Fc (Fig. 6). sgp130-Fc did not influence the response of BAF/gp130/LIFR/CNTFR cells to IL-3. Of note, the inhibitory effect of sgp130-Fc on LIF and OSM induced proliferation is very weak compared to its effect on the response stimulated by H-IL-6.

### sgp130-Fc inhibits sIL-6R dependent acute phase protein synthesis in HepG2 hepatoma ce/rs.

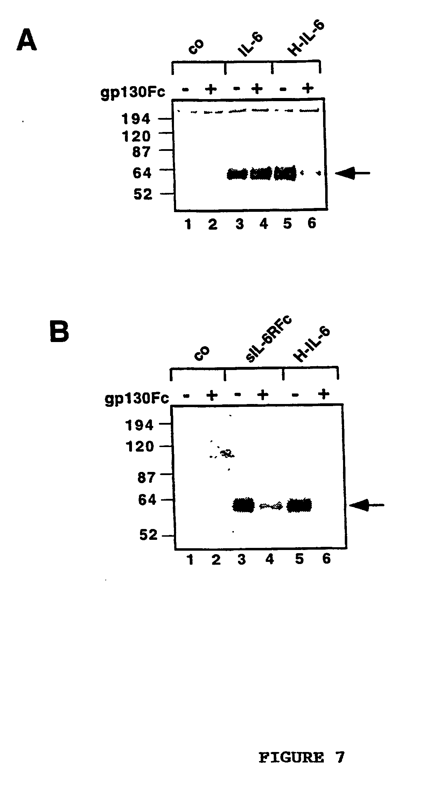

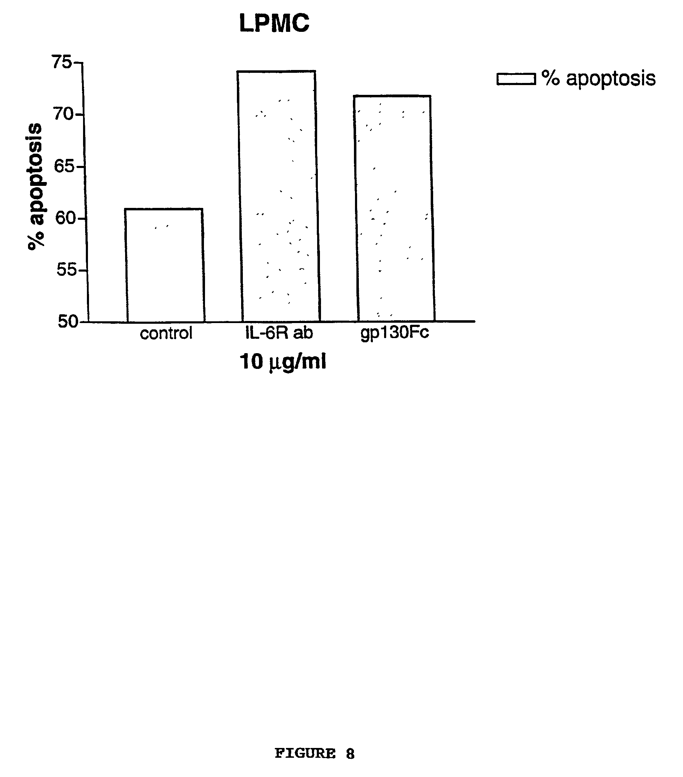

Human hepatoma cells HepG2 synthesize so called acute phase proteins such as haptoglobin and cd-antichymotrypsin in response to IL-6 (Gauldie et al., 1987) or IL-6/sIL-6R (Mackiewicz et al., 1992). HepG2 cells treated with IL-6 or HqL-6 in the absence or presence of sgp130-Fc were metabolically labeled for 4 hours, and subsequently the amount of α1-antichymotrypsin secreted into culture media was analyzed (Fig. 7A). sgp130-Fc had no effect on IL-6 induced synthesis of the protein, whereas the H-IL-6 induced synthesis of al-antichymotrypsin was strongly inhibited. These results underline our finding that sgp130-Fc specifically inhibits sIL-6R dependent responses and does not interfere with the cell surface expressed IL-6R.

Similar results were obtained with HepG2-IL-6 cells. These cells upon stable transfection of a human IL-6 cDNA lost surface expression of IL-6R. Although these cells secrete high amounts of IL-6 into their culture medium, they became completely unresponsive to IL-6. HepG2-IL-6 cells could, however, be stimulated by the complex of IL-6/sIL-6R [33]. In agreement with our results obtained with HepG2 cells, sIL-6R and H-IL-6 induced α1-antichymotrypsin synthesis in HepG2-IL-6 cells was strongly inhibited by sgp130-Fc.

### sgp130-Fc inhibits the antiapototic effect of sIL-6R on Crohn patients lamina propria cells.

Crohn's disease (MCD) is a chronic inflammatory disease of the gastrointestinal tract. It has been suggested that an activation of the mucosal immnue system in response to bacterial antigens with consecutive pathologic cytokine production plays a key pathogenic role. A neutralizing antibody directed against IL-6R was able to suppress the resistance of MCD patient lamina propria mononuclear cells (LPMC) against apoptosis. We wanted to analyze whether an antiapoptotic effect of IL-6 in this system is transduced via surface expressed IL-6R, or if the resistance of immnue competent cells against apoptosis is dependent of the presence of sIL-6R. LPMC were cultured in presence of neutralizing anti IL-6R antibody or sgp130-Fc under the conditions described in "Materials and Methods". Apoptotic cells were determined / detected as Annexin V positive, Propidiumiodide negative cells by FACS ananlysis. Untreated samples contained around 60 % apoptotic cells. Treatment with the IL-6R neutralizing antibody or sgp130-Fc resulted in an increase of apoptotic cells by 15 % and 13 %, respectively. The efficacy of sgp130-Fc points to an important role of sIL-6R for the resistance of LPMC against apoptosis. It is not likely that membrane expressed IL-6R contributes to the antiapoptotic effect, since antibody and sgp130-Fc treatment led to a similar increase in the number of apoptotic cells.

### EXAMPLE 2

### Materials, antibodies and cell lines

The gp130 neutralizing antibody GPX7 was a kind gift from Dr. Yasukawa (Tosoh, Tokyo, Japan). BAF/3 cells stably transfected with human gp130 and IL-6R cDNAs have been previously described (Fischer et al., 1997). A role for the immunoglobulin-like domain of the human IL-6 receptor: intracellular protein transport and shedding. *Eur J Blochem 263:438).* The preparation and characterization of the cell line HepG2-IL-6 has been described (Mackiewicz et al., 1992).

### Expression and purification of recombinant vIL-6

vIL-6 DNA was amplified by PCR and inserted into the mammalian expression plasmid pDC409 (Jostock et al., 1999) as a DNA 3' coding for a hexahistidine tagged protein using Sal I and Not I restriction sites. COS-7 cells were transfected using DEAE dextran, supernatants were collected after 5 days and purified at Ni-NTA agarose according to the manufactures instructions (Quiagen, Hilden, Germany). Column material was equilibrated with 50 mM phosphate buffer pH 7.5/500 mM NaCl/20 mM imidazole, after loading of culture supernatant the column was washed with equilibration buffer and eluted with 50 mM phosphate buffer pH 7.5/500 mM NaCl/100 mM imidazole. Eluted proteins were pooled and dialyzed against PBS at 4°C.

### Precipitation of vIL-6 with Fc fusion proteins.

COS-7 cells were transfected with the vIL-6 expression plasmid. 48 h after transfection cells were metabolically labeled for 8 h with 50 µCi/ml [³⁵S]-cysteine/methionine in cysteine/methionine-free medium (Gibco, Eggenstein, Germany). Supernatants were incubated for 2 h at 4°C with a rabbit antiserum raised against a bacterially expressed vIL-6 protein. Alternatively, supernatants were incubated with 2 pg/ml of human IL-6-Fc (Jostock et al., 1999), human sgp130-Fc or a human IL6R-Fc. The human IL-6R-Fc was constructed by fusing the cDNA coding for the extracellular portion of the human IL-6R to a cDNA coding for the human Fc portion of IgG1 (Jostock et al., 1999), using the restriction sites Sspl and EcoRV, respectively. The extracellular part of gp130 was fused to the Fc portion of a human IgG1 (Jostock et al., 1999), as a Xhol-EcoRI fragment. All Fc-proteins were transiently expressed in COS-7 cells and purified at protein A-Sepharose (Jostock et al., 1999). Immune complexes were precipitated with protein A-Sepharose, separated by SDS-PAGE and visualized by fluorography.

### BAF/3 cell profiferation assays

Proliferation of transfected BAF/3 cells was measured in 96-well microtitre plates. The cells were exposed to cytokines for 68 h and subsequently pulse-labeled with [³H]-thymidine for 4 h. Proliferation rates were measured by harvesting the cells on glass filters and determination of the incorporated radioactivity by scintillation counting. For each cytokine the proliferation assay was performed at least three times in triplicates.

### Analysis of phosphorylated STAT3 in hepatoma cells

HepG2-IL-6 cells were grown to confluency. 4 h prior to stimulation with cytokinee, cells were serum starved. After 15 rain of stimulation, cells were washed in PBS and lysed in laemmli buffer. Proteins were separated by 10 % SDS-PAGE electrophoresis and analyzed by Western blotting using the phospho-STAT3 (Y705) mAB Biolabs, Frankfurt, Germany).

### Isolation and culture of human vascular smooth muscle cells.

Smooth muscle cells (SMC) were obtained from pieces of human aortas obtained during aneurysm surgery (5 male donors, mean age 72 years) by courtesy of Dr. W. Schmiedt (Department of heart and thoracic surgery, University of Mairtz, Germany). Isolated media fragments were prepared (Ross, 1971) and smooth muscle cells were allowed to grow out from the media fragments which were kept in medium containing 1 ng/ml human recombinant basic fibroblast growth factor-β, 5 ng/ml human recombinant epidermal growth factor, 25 mg/L gentamycin and 1.25 mg/L amphotericin B at 37°C in 5% CO₂ in a humidified atmosphere. The purity of SMC's was evaluated by staining with an α-actin mAB (clone 1A4, Sigma, Deisenhofen, Germany). Medium was changed every 3 days. 24 h prior to experiments, SMC were grown in DMEM without any additives. Viability of cells was assayed using trypan-blue exclusion.

### RT-PCR of chemokine and cytokine mRNA and indirect immunofluorescence.

Total cellular RNA was isolated from confluent SMC cultures and reverse transcription of 1 pg of total RNA was conducted followed by PCR which was performed as follows: initial denaturation for 5 rain at 95°C then denaturation for 40 sec at 95°C, annealing for 1 min at 62°C and extension for 3 min at 72°C. PCR primers for MCP-1, IL-6, gp130 and GAPDH were as described (Klouche et al., 1999). PCR primers for the LIF-R have been described in ref. (Shimon et al., 1997). The PCR products were run on 1% agarose gels in 1 x TBE and stained with ethidium bromide. For immunocytochemical analysis, cells were fixed with 18.5% formaldehyde/12.5% glutaraldehyde for 2 h at room temperature and incubated with PE labeled gp130 specific mAB (clone AM64, IgG1; Pharmingen, San Diego, USA) overnight at 4°C followed by mounting and photography.

### Results

To express vIL-6 in mammalian cells, the cDNA was cloned into an expression vector (Fischer et al., 1997) which added a hexahistidine tag to the COOH terminus of the protein. The protein was purified to near homogeneity via Ni-affinity chromatography. Upon SDS-PAGE analysis of the purified protein a double band of 24-26 kDa is visible which most likely reflects differentially glycosylated forms of vIL-6.
The metabolically ³⁵S-labeled vIL-6 protein could be precipitated from the supernatant of transfected cells with a rabbit antiserum raised against a bacterially expressed vIL-6 protein. To test for physical interaction with receptor subunits of the IL-6 receptor system, the ³⁵S-vIL-6 protein was incubated with Fc fusion proteins containing IL-6, the extracellular portion of the IL-6R, and the extracellular portion of gp130. Protein complexes were precipitated with protein A Sepharose. Neither IL-6-Fc nor IL-6R-Fc interacted with vIL-6. In contrast, sgp130-Fc coprecipitated the vIL-6 protein indicating binding of vIL-6 to the extracellular portion of gp130. The specificity of the interaction of the Fc proteins with their cognate ligands is demonstrated as follows. Human metabolically S-labeled IL-6 is precipitated with IL-6R-Fc as a broad band reflecting different glycosylation states of recombinantly expressed human IL-6 (Schiel et al., 1990). No IL-6 was precipitated with IL-6-Fc and sgp130-Fc. In contrast, a fusion protein of sIL-6R and IL-6 (Hyper-IL-6), which has been shown to directly stimulate gp130 (Fischer et al., 1997), is precipitated with sgp130-Fc but not with IL-6-Fc or IL-6R-Fc.
We next asked whether vIL-6 bound to gp130 at the same site as the IL-6/sIL-6R complex. Precipitation of metabolically labeled vIL-6 by sgp130-Fc was performed in the presence or absence of human IL-6 and Hyper-IL-6. Binding of vIL-6 to sgp130-Fc was not affected by an excess of human IL-6. Precipitation of vIL-6 by sgp130-Fc, however, was completely blocked in the presence of Hyper-IL-6. This experiment clearly demonstrated that vIL-6 and Hyper-IL-6 competed for the same binding site on human gp130.
Having shown a direct interaction of vIL-6 with gp130, we next asked whether the IL-6R was needed for biologic activity of vIL-6. We made use of the IL-3 dependent pre-B cells BAF/3 which do not express gp130 and are therefore not responsive to IL-6 or IL-6/siL-6R. BAF/3 cells stably transfected with human gp130 cDNA or human gp130 and IL-6R cDNAs, however, in the absence of IL-3 grow in response to IL-6/sIL-6R or IL-6, respectively (Vollmer et al., 1999). BAF/3 cells transfected with gp130 cDNA (BAF-130 cells) grow in response to increasing amounts of Hyper-IL-6. When the same cells were stimulated with IL-6, no growth of the cells was observed. Cells stimulated with vIL-6, however, showed dose dependent proliferation. In agreement with the lower effectivity of binding to gp130, vIL-6 proved to have a 20-50 fold lower specific biologic activity than Hyper-IL-6. On BAF/3 cells transfected with gp130 and IL-6R cDNAs (BAF-130/IL-6R cells) proliferation was observed in response to IL-6, Hyper-IL-6 and vIL-6. The specific biologic activity of IL-6 was 100-times higher than that of vIL-6. BAF-130/IL-6R cells are more sensitive to IL-6 than to Hyper-IL-6 since they express more IL-6R than gp130 (Vollmer et al., 1999).
We next examined whether sgp130-Fc inhibited the biologic activity of vIL-6. We found that addition of sgp130-Fc to BAF-130 cells stimulated with Hyper-IL-6 or vIL-6 led to a dose dependent inhibition of cytokine induced proliferation. In contrast, in BAF-130/IL-6R cells stimulated with human IL-6, sgp130-Fc did not inhibit IL-6 induced proliferation. The lack of inhibition of human IL-6 may indicate that there is no free access of the sgp130-Fc fusion protein to the membrane associated IL-6/IL-6R complex. Therefore, the sgp130-Fc fusion protein specifically inhibits vIL-6 but not human IL-6 acting via the membrane bound IL-6R.
Cytokine stimulation of gp130 leads to gp130 dimerization, subsequent phosphorylation of gp130, activation of JAK kinases and tyrosine phosphorylation of STAT3. Phosphorylated STAT3 dimerizes and translocates into the nucleus where it binds to specific DNA sequences and acts as a transcription factor (Taga et al., 1997). We therefore asked whether cellular stimulation by vIL-6 was also mediated by gp130 activation. Addition of a neutralizing mAB directed against gp130 completely blocked proliferation of BAF-130 cells induced by Hyper-IL-6 and by vIL-6. The same gp130 neutralizing mAB did not influence proliferation of untransfected BAF/3 cells induced by IL-3. These results indicate that both, Hyper-IL-6 and vIL-6 acted via gp130 activation.
To demonstrate that stimulation of cells with vIL-6 led to intracellular phosphorylation and activation of STAT3 we employed HepG2-IL-6 cells. HepG2 cells upon stable transfection of a human IL-6 cDNA lost surface expression of IL-6R and therefore became completely unresponsive to IL-6. HepG2-IL-6 cells could, however, be stimulated by the complex of IL-6/sIL-6R (Mackiewicz et al., 1992). In un-stimulated and IL-6 stimulated HepG2-IL-6 cells, no phosphorylation of STAT3 was detectable. In contrast, incubation of cells in the presence of Hyper-IL-6 or vIL-6 resulted in phosphorylation of STAT3. This phosphorylation of STAT3 could completely be blocked by a neutralizing mAB directed against gp130. These experiments demonstrated that activation by vIL-6 was not mediated by the cell bound IL-6R. Furthermore, our results suggest that vIL-6 and Hyper-IL-6 use the same cellular activation mechanism via gp130.
The Kaposi sarcoma is characterized by new vessel formation and proliferation of spindle cells, "KS-cells", which are associated with endothelial cells, fibroblasts and inflammatory cells. Historically, proliferating spindle cells were considered to be derived from endothelial cells, but more recently, both types of mesenchymal cells, endothelial and smooth muscle cells, have been proposed as potential progenitors. Immunohistochemical staining and Northern blot analysis revealed a coexpression of antigens specific for endothelial and smooth muscle cells (Kaaya et al., 1995). In earlier studies it was observed that smooth muscle cells could only be stimulated with IL-6 in the presence of sIL-6R (Klouche et al., 1999).
We therefore studied the stimulation of human primary smooth muscle cells by vIL-6. In human smooth muscle cells the mRNA expression of IL-6, of the monocyte attracting chemokine MCP-1 and of gp130 are upregulated in a time dependent fashion upon stimulation by vIL-6. Both, induction of MCP-1 as well as virally encoded chemokines may be involved in the characteristic attraction and presence of inflammatory cells in KS.
Besides gp130, the receptor subunit LIF-R is shared by cytokines of the IL-6 family such as LIF, CNTF, OSM and CT-1 (Taga et al., 1997). We therefore asked whether the expression of LIF-R mRNA was also modulated by vIL-6 and Hyper-IL-6. Treatment of smooth muscle cells with Hyper-IL-6 and vIL-6 led to an upregulation of LIF-R mRNA as measured by the appearance of a 359 bp DNA band upon RT-PCR, indicating that stimulation of smooth muscle cells by vIL-6 resulted in increased expression of the co-receptor for the IL-6 family members LIF, CNTF, OSM and CT-1.
Furthermore, we found that gp130 cell surface expression is strongly increased after treatment of the cells with vIL-6 and vIL-6 induced proliferation of the cells. Notably, expression of gp130 preceeded that of IL-6, indicating the induction of an autocrine stimulation loop by vIL-6. Treatment with human IL-6 in the absence of sIL-6R had no effect on MCP-1, IL-6 and gp130 expression and on proliferation (Klouche et al., 1999). We conclude from these data that vIL-6, like the IL-6/sIL-6R,. complex induces a proinflammatory state in human smooth muscle cells.

### REFERENCES

Aruffo A, Kolanus W, Walz G, Fredman P, Seed B, 1991, CD62/P-selection recognition of myeloid and tumor cell sulfatides, Cell, 67(1):35-44.
Ashkenazi, A., and Chamow, S. M. (1997) Immunoadhesins as research tools and therapeutic agents. Curr Opin. Immunol. 9, 195-200.
Ausubel et al., Current Protocols in Molecular Biologic supra. Interscience. N.Y.. (1987-1998).
Burger, R., F. Neipel, B. Fleckenstein, R. Savino, G. Ciliberto, J. R. Kalden, and M. Gramatzki. 1998. Human herpesvirus type 8 interleukin-6 homologue is functionally active on human myeloma cells. Blood, 91:1858.
Chamow, S. M., and Ashkenazi, A. (1996) Immunoadhesins: principles and application. Trends Biotechnol 14, 52-60.
Cunningham BC, Wells JA (1989a), High-resolution epitope mapping of hGH-receptor interactions by alanine-scanning mutagenesis, Science, 244(4908):1081-5.
Cunningham BC, Jhurani P, Ng P, Wells JA, (1989b), Receptor and antibody epitopes in human growth hormone identified by homolog-scanning mutagenesis, Science; 243(4896):1330-6.
Fanslow, W. C., Anderson, D. M., Grabstein, K. H., Clark, E. A., Cosman, D., and Armitage, R. J. (1992) Soluble forms of CD40 inhibit biologic responses of human B cells. J. Immunol 149, 655-660.
Fischer, M., Goldschmitt, J., Peschel, C., Kallen, K. J., Brakenhoff, J. P. J., Wollmer, A., Grötzinger, J., and Rose-John, S. (1997) A Designer Cytokine with High Activity on Human Hematopoietic Progenitor Cells. Nature Biotech. 15,142-145.
Galliard, J.-P., Liautard, J., Klein, B., and Brochier, J. (1997) Major role of the soluble interleukin-6/interleukin-6 receptor complex for the proliferation of interleukin-6-dependent human myeloma cell lines. Eur. J. Immunol. 27, 3332-3340.
Gauldie, J., Richards, C., Harnish, D., Lansdorp, P., and Baumann, H. (1987) Interferon beta 2/B-cell stimulatory factor type 2 shares identity with monocyte-derived hepatocyte-stimulating factor and regulates the major acute phase protein response in liver cells. Proc. Natl. Acad. Sci. USA 84, 7251-7255.
Giri, J. G., Ahdieh, M., Eisenman, J., Shanebeck, K., Grabstein, K., Kumaki, S., Namen, A., Park, L. S., Cosman, D., and Anderson, D. (1994) Utilization of the beta and gamma chains of the IL-2 receptor by the novel cytokine IL-15. EMBO J. 13, 2822-2830.
Greenberg AS, Nordan RP, McIntosh J, Calvo JC, Scow RO, Jablons D, (1992), Interleukin 6 reduces lipoprotein lipase activity in adipose tissue of mice in vivo and in 3T3-L1 adipocytes: a possible role for interleukin 6 in cancer cachexia, Cancer Res; 52(15):4113-6.
Hibi M, Murakami M, Saito M, Hirano T, Taga T, Kishimoto T (1990), Molecular cloning and expression of an IL-6 signal transducer, gp130, Cell ; 63(6):1149-57.
Horsten, U., Schmitz-Van de Leur, H., Müllberg, J., Heinrich, P. C., and Rose-John, S. (1995) The membrane distal half of gp130 is responsible for the formation of a ternary complex with IL-6 and the IL-6 receptor, FEBS Lett.; 360, 43-46.
Jilka RL, Hangoc G, Girasole G, Passeri G, Williams DC, Abrams JS, Boyce B, Broxmeyer H, Manolagas SC (1992)] Increased osteoclast development after estrogen loss: mediation by interleukin-6, Science; 257(5066):88-91.
Jostock, T., BJinn, G., Renne, C., Kallen, K.-J., Rose-John, S., and Müllberg, J. (1999) Immunoadhesins of IL-6 and Hyper-IL-6.J. Immunol. Meth.; 223, 171-183.
Kaaya, E. E., C. Parravicini, C. Ordonez, R. Gendelman, E. Berti, R. C. Gallo, and P. Biberfeld. 1995. Heterogeneity of spindle cells in Kaposi's sarcoma: comparison of cells in lesions and in culture. J. Acquit. Immune Doric. Syndr. Hum. Retrovirol.; 10: 295.
Kallen, K. J., Galle, P., R., and Rose-John, S. (1999) IL-6 dependent biology and therapy: where do we stand and what are the options? Exp. Opin Invest. Drugs; 8, 1-23.
Kallen, K. J., Grötzinger, J., Lelibvre, E., Veilmet, P., Aasland, D., Renne, C., Mdllberg, J., Meyer zum Büschenfelde, K.H., Gascan, H., and Rose-John, S. (1999) Receptor recognition sites of cytokines are organized as exchangeable moduls: transfer of the LIFR binding site from CNTF to IL-6.J. Biol. Chem. 274, 11859-11867.
Kallen, K.-J., Meyer zum Büschenfelde, K. H., and Rose-John, S. (1997) The therapeutic potential of interleukin-6 hyperagonists and antagonists. Exp. Opin. Invest. Drugs 6, 237-266.
Klouche, M., Bhakdi, S., Hemmes, M., and Rose-John, S. (1999) Novel Path of activation of primary human smooth muscle cells: upregulation of gp130 creates an autocrine activation loop by IL-6 and its soluble receptor. J. Immunol.; 163, 4583-4589.
Krebs, B., Griifin, H., Winter, G., and Rose-John, S. (1998) Recombinant Human scFv Antibodies Recognizing Human Interleukin-6: Specific Targeting of Cytokine Secreting Cells. J. Biol. Chem.; 273, 2858-2865.
Laemmli, U. K. (1970) Cleavage of structural proteins during the assembly of the head of bacteriophage T4 .Nature 227, 680-685.
Lust, J. A., Donovan, K. A., Kline, M, P., Greipp, P. R., Kyle, R. A., and Maihle, N. J. (1992) Isolation of an mRNA encoding a soluble form of the human interleukin-6 receptor. Cytokine; 4,96-100.
Mackiewicz, A., Schooltink, H., Heinrich, P. C., and Rose-John, S. (1992) Complex of soluble human IL-6-receptor/IL-6 up-regulates expression of acute-phase proteins. J. Immunol. 149, 2021-2027.
Mackiewicz, A., Wiznerowicz, M., Roeb, E., Karczewska, A., Nowak, J., Heinrich, P. C., and Rose-John, S. (1995) Soluble intedeukin 6 receptor is biologically active in vivo. Cytokine 7, 142-149.
Maione, D., Di Carlo, E., Li, W., Musiani, P., Modesti, A., Peters, M., Rose-John, S., Della Rocca, C., Tripodi, M., Lazzaro, D., Taub, R., Savino, R., and Ciliberto, G. (1998) Coexpression of IL-6 and soluble IL-6R causes nodular regenerative hyperplasia and adenomas of the liver. EMBO J 17, 5588-5597.
März, P., Cheng, J.-C., Gadlent, R. A., Patterson, P., Stoyan, T., Otten, U., and Rose-John, S. (1998) Sympathetic Neurons can produce and respond to Interleukin-6.Proc. Natl. Acad. Sci. USA 95,3251-3256.
März, P., Herget, T., Lang, E., Otten, U., and Rose-John, S. (1997) Activation of gp130 by IL-6/soluble IL-6 Receptor Induces Neuronal Differentiation. Eur. J. Neurosci. 9, 2765-2773.
März, P., Otten, U., and Rose-John, S. (1999) Neuronal Activities of IL-6 Type Cytokines often Depend on Soluble Cytokine Receptors. Eur. J. Neurosci. 11, 2995-3004.
McMahan, C. J., Slack, J. L, Mosiey, B., Cosman, D., Lupron, S. D., Brunton, L. L., Grubin, C. E., Wignail, J. M, Jenkins, N. A., Brannan, C. L, Copeland, N. G., Huebner, K., Croce, C. M., Cannizzarro, L. A., Benjamin, D., Dower, S. K., Spriggs, M K., and Sims, J. E. (1991) A novel IL-1 receptor, cloned from B cells by mammalian expression, is expressed in many cell types. EMBO J. 10, 2821-2832.
Molden, J., Y. Chang, Y. You, P. S. Moore, and M. A. Goldsmith. 1997. A Kaposi's Sarcoma-associated Herpesvirus-encoded Cytokine Homolog (vIL-6) Activates Signaling through the Shared gp130 Receptor Subunit. J. Biol. Chem.; 272:19625.
Moore, P. S., C. Boshoff, R A. Weiss, and Y. Chang. 1996. Molecular mimicry of human cytokine and cytokine response pathway genes by KSHV. Science; 274 (5293): 1739-44.
Müllberg, J., Schoolfink, H., Stoyan, T., Gunther, M., Graeve, L., Buse, G., Mackiewicz, A., Heinrich, P. C., and Rose-John, S, (1993) The soluble interleukin-6 receptor is generated by shedding. Eur. J. Immunol. 23, 473-480.
Narazaki, M., Yasukawa, K., Saito, T., Ohsugi, Y., Fukui, H., Koishihara, Y., Yancopoulos, G. D., Taga, T., and Kishimoto, T. (1993) Soluble forms of the interleukin-6 signal-transducing receptor component gp130 in human serum possessing a potential to inhibit signals through membrane-anchored gp130. Blood, 82, 1120-1126.
Nishimoto N, Kishimoto T, Yoshizaki K, (1999), Anticytokine therapy in autoimmune diseases, Intern Med; 38(2): 178-82.
Özbek, S., Grötzinger, J., Krebs, B., Fischer, M., Wollmer, A., Jostock, T., Müllberg, J., and Rose-John, S. (1998) The Membrane Proximal Cytokine Receptor Domain of the Human Intedeukin-6-Receptor is Sufficient for Ligand Binding but not for gp130 Association. J. Biol. Chem.; 273, 21374-21379.
Peters, M, Schirmacher, P., Goldschmitt, J., Odenthal, M., Peschel, C., Dienes H. P., Fattori, E., Ciliberto, G., Meyer zum Büschenfelde, K. H., and Rose-John S. (1997) Extramedullary expansion of hematopoietic progenitor cells in IL-6/sIL-6R double transgenic mice. J. Exp. Med.; 185, 755-766.
Peters, M., Jacobs, S., Ehiers, M,, Volliner, P., Müllberg, J., Wolf, E., Brem, G., Meyer zum Büschenfelde, K. H., and Rose-John, S. (1996) The function of the soluble interleukin 6 (IL-6) receptor in vivo: sensitization of human soluble IL-6 receptor transgenic mice towards IL-6 and prolongation of the plasma half-life of IL-6.J. Exp. Med.; 183, 1399-1406.
Peters, M., Müler, A., and Rose-John, S. (1998) Interleukin-6 and soluble Interleukin-6 Receptor: Direct Stimulation of gp130 and Hematopoiesis. Blood; 92, 3495-3504.
Romano, M., Sironi, M., Toniatti, C., Polentarutti, N., Fruscella, P., Ghezzi, P., Faggioni, R., Luini, W., van Hinsbergh, V., Sozzani, S., Bussolino, F., Poll, V., Ciliberto, G., and Mantovani, A. (1997) Role of IL-6 and its soluble receptor in induction of chemokines and leukocyte recruitment. Immunity S.; 315-325.
Rose-John, S., and Heinrich, P. C. (1994) Soluble receptors for cytokines and growth factors: generation and biological function. Biochem. J. 300,281-290.
Ross, R. 1971. The smooth muscle cell. II. Growth of smooth muscle in culture and formation of elastic fibers. J Cell Biol; 50: 172.
Sambrook et al., Molecular Cloning: A Laboratory- Manual. second Ed., Cold Spring harbor, NY, 1989.
Sharkey AM, Dellow K, Blayney M, Macnamee M, Charnock-Jones S, Smith SK, (1995), Stage-specific expression of cytokine and receptor messenger ribonucleic acids in human preimplantation embryos , Biol Reprod Oct;53 (4):974-81.
Schiel, X., S. Rose-John, G. Dufhues, H. Sohooltink, V. Gross, and P. C,. Heinrich. 1990. Microheterogeneity of human interleukin 6 synthesized by transfected NIH/3T3 cells: comparison with human monocytes, fibroblasts and endothelial cells. Eur. J. Immunol.; 20: 883.
Shimon, L, X. Yan, D. W. Ray, and M. Shlomo. 1997. Cytokine dependent gp130 stimulation receptor subunit regulates human fetal pituitary adrenocorticotropin hormone and growth hormone secretion. J. Clin. Invest.; 100: 357.
Schirmacher, P., Peters, M., Ciliberto, G., Fattori, E., Lotz, J., Meyer zum Büschenfelde, K. H., and Rose-John, S. (1998) Hepatocellular Hyperplasia, Plasmacytoma Formation, and Extracellular Hematopoiesis in Interleukin (IL)-6/Soluble IL-6 Receptor Double-Transgenic Mice, Am. J. Pathol.; 153, 639-648.
Strassmann G, Fong M, Kenney JS, Jacob CO, (1992), Evidence for the involvement of interleukin 6 in experimental cancer cachexia, J Clin Invest.; 89(5): 1681-4.
Sui, X., Tsuji, K., Tajima, S., Tanaka, R., Muraoka, K., Ebihara, Y., Ikebuchi, K., Yasukawa, K., Taga, T., Kishimoto, T., and Nakahata, T. (1996) Erythropoietin-independent erythrocyte production: signals through gp130 and c-kit dramatically promote erythropoiesis from human CD34+ cells. J. Exp. Med; 183, 837-845.
Sui, X., Tsuji, K., Tanaka, R, Tajima, S., Muraoka, K., Ebihara, Y., Ikebuchi, K., Yasukawa, K., Taga, T., Kishimoto, T., and Nakahata, T. (1995) gp130 and c-Kit signalings synergize for ex vivo expansion of human primitive hemopoietic progenitor cells. Proc. Natl. Acad. Sci. USA; 92, 2859-2863.
Taga, T., and Kiskmoto, T. (1997a) gp 130 and the Interleukin-6 Family of Cytokines. Annal Rev. Immunol. 15, 797-819.
Taga and Kishimoto (1997b) gp130 and the interleukin-6 family of cytokines. Annu. Rev. Immunol. 15,797-819.
Taga, T., Hibi, M., Hirata, Y., Yamasaki, K., Yasukawa, K., Marsuda, T., Hirano, T., and Kishimoto, T. (1989) Intedeukin-6 triggers the association of its receptor with a possible signal transducer, gp130. Cell, 58,573-581.
Taga, T., Hibi, M., Hirata, Y., Yawata, H., Natsuka, S., Yasukawa, K., Totsuka, T., Yamasaki, K., Hirano, T., and Kishimoto, T. (1989) Interleukin-6 receptor and a unique mechanism of its signal transduction. Cold Spring Harb Syrup Quant Biol 54 Pt 2, 713-722.
Tajima, S., Tsuji, K., Ebihara, Y., Sui, X., Tanaka, R, Muraoka, K., Yoshida, M., Yamada, K., Yasukawa, K., Taga, T., Kishimoto, T., and Nakahata, T. (1996) Analysis of interleukin-6 receptor and gp130 expressions and proliferative capability of human CD34+ cells. J. Exp. Med. 184, 1357-1364.
Tamura, T., Udagawa, N., Takahashi, N., Miyaura, C., Tanaka, S., Yamada, Y., Koishihara, Y., Ohsugi, Y-, Kumaki, K., Taga, T., Kishimoto, T., and Suda, T. (1993) Soluble interleukin-6 receptor triggers osteoclast formation by interleukin-6. Proc. Natl. Acad. Sci. USA 90, 11924-11928.
Udagawa, N., Takahashi, N., Katagiri, T., Tamura, T., Wada, S., Findlay, D. M, Martin, T. J., Hirota, H., Taga, T., Kishimoco, T., and Suda, T. (1995) Interleukin (IL)-6 induction of osteoctast differentiation depends on IL-6 receptors expressed on osteoblastic cells but not on osteoclast progenitors. J. Exp. Med. 182, 1461-1468.
van Dam, M., Müllberg, J., Schooltink, H., Stoyan, T., Brakenhoff, J. P., Graeve, L., Heinrich, P. C., and Rose-John, S. (1993) Structure-function analysis of intedeukin-6 utilizing human/murine chimeric molecules. Involvement of two separate domains in receptor binding. J. Biol. Chem. 268, 15285-15290.
van Oers MH, van Zaanen HC, Lokhorst HM, (1993) Interleukin-6, a new target for therapy in multiple myeloma? Ann. Hematol.; 66(5): 219-23.
van Snick, J. (1990) Interleukin-6: an overview. Annu. Rev. Immunol 8, 253-279.
Van Zee, K. J., Kohno, T., Fischer, E., Rock, C. S., Moldawer, L. L., and Lowry, S. F. (1992) Tumor necrosis factor soluble receptors circulate during experimental and clinical inflammation and can protect against excessive tumor necrosis factor alpha in vitro and in vivo. Proc. Natl. Acad. Sci. USA 89, 4845-4849.
Vollmer, P., B. Oppmann, N. Voltz, M. Fischer, and S. Rose-John. 1999. A role for the immunoglobulin-like domain of the human IL-6 receptor: intracellular protein transport and shedding. Eur J Blochem; 263: 438.
Yoshizaki K, Kuritani T, Kishimoto T, (1992) Interleukin-6 in autoimmune disorders, Semin Immunol.; 4(3): 155-66.

## Claims

1. Pharmaceutical composition containing a fusion molecule comprising two soluble gp130 molecules.

2. The pharmaceutical composition of claim 1, wherein two soluble gp130 molecules are fused together in tandem by a flexible loop.

3. The pharmaceutical composition of claim 1 or 2, wherein at least one of the two soluble gp130 molecules is the entire extracellular domain of gp130.

4. The pharmaceutical composition of claim 1 or 3, wherein the two soluble gp130 molecules are linked to each other through a simple covalent bond.

5. The pharmaceutical composition of anyone of claims 2 to 4, wherein the two soluble gp130 molecules are linked to each other through a flexible peptide linker.

6. The pharmaceutical composition of claim 1, wherein the two soluble gp130 molecules are fused to the Fc domain of the IgG proteins, so that it is expressed as disulfide-linked homodimer.

7. A pharmaceutical composition containing a DNA molecule encoding a molecule as defined in claim 1.

8. A pharmaceutical composition containing an expression vector containing a DNA molecule as defined in claim 1.

9. Use of a compound as defined in anyone of claims 1 to 8 for the preparation of a medicament for the treatment and/or prevention of multiple myeloma, autoimmune diseases, osteoporosis, cachexia or bacterial and viral infections.

## Patentansprüche

1. Pharmazeutische Zusammensetzung enthaltend ein Fusionsmolekül, dass zwei lösliche gp 130 Moleküle umfasst.

2. Pharmazeutische Zusammensetzung nach Anspruch 1, wobei zwei lösliche gp 130 Moleküle mit einer flexiblen Schleife zu einem Tandem fusioniert sind.

3. Pharmazeutische Zusammensetzung nach Anspruch 1 oder 2, wobei zumindest eines der zwei löslichen gp 130 Moleküle eine vollständige, extrazelluläre Domäne von gp 130 ist.

4. Pharmazeutische Zusammensetzung nach Anspruch 1 oder 3, wobei die zwei löslichen gp 130 Moleküle durch eine einfache kovalente Bindung miteinander verbunden sind.

5. Pharmazeutische Zusammensetzung nach einem der Ansprüche 2 bis 4, wobei die zwei löslichen gp 130 Moleküle durch einen flexiblen Peptidlinker miteinander verbunden sind.

6. Pharmazeutische Zusammensetzung nach Anspruch 1, wobei die zwei löslichen gp 130 Moleküle an die Fc Domäne der IgG Proteine fusioniert sind, so dass sie als Disulfid-verbundener Homodimer exprimiert werden.

7. Pharmazeutische Zusammensetzung enthaltend ein DNA Molekül, dass ein Molekül, wie in Anspruch 1 definiert, kodiert.

8. Pharmazeutische Zusammensetzung enthaltend einen Expressionsvektor, der ein DNA Molekül, wie in Anspruch 1 definiert, enthält.

9. Verwendung einer Verbindung, wie in einem der Ansprüche 1 bis 8 definiert, für die Herstellung eines Medikaments zur Behandlung und/oder Verhinderung von multiplem Myelom, Autoimmunkrankheiten, Osteoporose, Kachexia oder bakteriellen und viralen Infektionen.

## Revendications

1. Composition pharmaceutique contenant une molécule hybride comprenant deux molécules solubles de gp130.

2. Composition pharmaceutique selon la revendication 1, **caractérisée en ce que** deux molécules solubles de gp130 sont fusionnées en tandem par une boucle flexible.

3. Composition pharmaceutique selon la revendication 1 ou 2, **caractérisée en ce qu'**au moins une des deux molécules solubles de gp130 est le domaine extracellulaire entier de la gp130.

4. Composition pharmaceutique selon la revendication 1 ou 3, **caractérisée en ce que** les deux molécules solubles de gp130 sont liées l'une à l'autre par une simple liaison covalente.

5. Composition pharmaceutique selon l'une quelconque des revendications 2 à 4, **caractérisée en ce que** les deux molécules solubles de gp130 sont liées l'une à l'autre par un liant peptidique flexible.

6. Composition pharmaceutique selon la revendication 1, **caractérisée en ce que** les deux molécules solubles de gp130 sont fusionnées au domaine Fc des protéines IgG, de façon à être exprimées comme un homodimère lié par pont disulfure.

7. Composition pharmaceutique contenant une molécule d'ADN encodant une molécules telle que définie dans la revendication 1.

8. Composition pharmaceutique contenant un vecteur d'expression contenant une molécule d'ADN telle que définie dans la revendication 1.

9. Utilisation d'un composé tel que défini dans l'une quelconque des revendications 1 à 8 pour la préparation d'un médicament pour le traitement et/ou la prévention des myélomes multiples, des maladies autoimmunes, de l'ostéoporose, de la cachexie ou des infections bactériennes ou virales.
